# EUROPEAN PATENT SPECIFICATION

(11) **EP 1 512 409 B1**
(45) Date of publication and mention of the grant of the patent: **08.11.2006**
(21) Application number: 04255216.6
(22) Date of filing: 27.08.2004
(51) Int. Cl.: A61K 36/00, A01N 65/00, A61P 33/14

(54) **Composition against infestment of lice**
Mittel gegen Lausbefall
Agent pour le traitement des poux de la tête

(30) Priority: 04.09.2003 GB 0320664
(43) Date of publication of application: 09.03.2005
(73) Proprietor: Dolisznyj, Helen, Penkridge, Staffordshire ST19 5NT (GB)
(72) Inventor: Dolisznyj, Helen, Penkridge, Staffordshire ST19 5NT (GB)
(74) Representative: Stanley, Michael Gordon

(56) References cited:
- CH-A- 688 787
- US-B1- 6 342 253

## Description

### Field

This invention relates to a composition useful for as a hair cleaning agent particularly for the removal of insects and their eggs, and is particularly for use on people as a treatment for headlice and their eggs.

### Background of Invention

The problem of headlice persists in both developed and developing countries and is particularly prevalent amongst school children amongst whom headlice are easily spread by hair contact between individuals. The problem is becoming worse as headlice build up a resistance to many commercially available non-prescription treatments, that is treatments available across the counter without a doctor's prescription. Another reason for the lack of success of existing treatments is that they fail to destroy the eggs and that several treatments must take place over the life cycle of the headlice as existing eggs hatch.

Furthermore such treatments are expensive and must be carefully applied and may take a prolonged period of time to be effective, sometimes hours. They are therefore very difficult to apply properly especially when treating the heads of children who might have difficulty in remaining still over the required time period and may get some of the treatment composition in their eyes.

Shampoos for the control of headlice are known, for example US 5866 152 which discloses a shampoo which contains insecticides as the active ingredients e.g pyrethroid compounds and pyriproxyfen. Other compositions have been formulated which contain natural products for example EP1048293. In this document there is described a composition containing mixtures of essential oils and /or terpenoids. US 6342 253 B1 describes a treatment for headlice which includes a mixture of essential oils. EP 0945066 discloses a natural pesticide for use with plants which uses both garlic extract and essential oils. CH688787 discloses compositions comprising essential or etheric oils, which may further contain aq extracts of plants for use against infestation of parasites, including lice.

The present invention provides a hair wash or hair cleaner that contains no chemical insecticides, and whose active ingredients are derived from natural sources and which is effective within a short time period.

### Statements of Invention

According to the invention there is provided an aqueous composition for use as a hair wash for the treatment of lice and their eggs and which includes as its active ingredients at least one essential oil, characterised in that the composition further includes an infusion of: dried peppermint leaves, a tea, and garlic.

By tea is meant the leaves of the Chinese, Indian, Srilankan, Kenyan, or any other variety thereof either singly or mixed.

The infusion of tea may be an infusion of a mixture of tea and oswego tea ( bergamot) commonly known as Earl Gray tea.

Preferably the composition contains an essential oil comprising at least one of citronella, tea tree oil, and eucalyptus oil and which more preferably comprises less than 3% of the total composition by weight. The preferred essential oils are citronella for the eradication of lice and a mixture of citronella and tea tree oil, preferably a 50/50 mixture, for the eradication of eggs.

In a typical composition the extracted active ingredients infused into the water comprise less than 0.5% of the total composition by weight.

The composition may further comprise acetic acid present up to a maximum of 2.5% by weight.

The composition may further comprise colourants, conditioning agents, moisturisers, perfumes, surfactants and thickening agents. The thickening agent may comprise carboxyl methylcellulose, or polyglycols, or preferably guagum,

According to yet another aspect of the invention there is provided a method of manufacture of a composition for the treatment of headlice including the steps of making an infusion of peppermint leaves, tea and garlic in boiling water and allowing to cool, and then mixing the cooled infusion with at least one essential oil, surfactants and a thickening agent to form a gel.

The essential oil may comprise at least two oils mixed prior to addition to the infusion. The oil surfactants and thickening agents, and other additives may be added to infusion together with an aqueous solution of acetic acid.

### Specific Example

The invention will be described by way of example and with reference to the following example of an aqueous composition which is intended for use as a shampoo for application to the hair or skin of a human, or other animal, for the treatment of headlice.

The composition is mixed in two stages.

In the first stage a mixture containing

| | |
|---|---|
| 180 litres | water |
| 600 g | dried peppermint leaves( Mentha piperita) |
| 500 g | Earl Gray tea |
| 1.45 kg | fresh garlic bulbs |

is boiled for 5 hours and then allowed to stand and cool over several hours, for example overnight. The cold liquor is the poured off the remnants and split into two 90 litre batches.

The infusion contains: a) a mixture of oils including menthol, cineol, menthene, limonene, and others from the peppermint leaves , b) alkaloids such as caffeine, polyphenols including flavonols, and phenolic acids such as gallic acid, found in tea
and c) allicin and other known compounds from the garlic.

In the second stage each 90 litres of liquor is then mixed as follows:

| | |
|---|---|
| 90 litres | liquor |
| 90 litres | 4.5 % dilute aqueous acetic acid |
| 5 litres | essential oil |
| 1.5 litres | detergent |

The whole was mixed for 3 hours with between 1.0-1.2 kg (approx 0.5-0.6% by weight) of guagum thickening agent (Meypro Guar) being added over the time period to produce a thin paste composition suitable for the treatment of hair of humans or animals to remove headlice simply by using the composition as a hair wash.

Colourants, moisturiser and other additives may be added at this stage.

The paste may be bottled or stored in flexible containers and used as a hair cleaner.

The preferred essential oils are tea tree oil, a 50/50 mix of tea tree oil and eucalyptus oil, citronella, a 50/50 mix of citronella and tea tree oil, and eucalyptus oil.

The hair cleaner may be used as a mid-wash treatment in a similar manner to a hair conditioner.

### Tests against lice & louse eggs

Samples containing different essential oils were made up and tested for efficacy against lice and their eggs under laboratory conditions. The samples contained the following essential oils:

| | |
|---|---|
| Sample 1 | tea tree & eucalyptus 50/50 |
| Sample 2 | eucalyptus |
| Sample 3 | citronella |
| Sample 4 | tea tree |
| Sample 5 | tea tree & citronella 50/50 |

Louse eggs were obtained from the culture colony maintained by Insect Research and Development Ltd of Cambridge.

In the tests for efficacy against adult lice, approximately equal numbers of male and female lice were used. The lice were fed on the morning of the test and allowed four hours to recover in which time they excreted excess water imbibed during feeding. Lice were counted into batches that were each provided with squares of open mesh nylon gauze on which to stand and each batch allocated to a 30 mm Petri dish.

Louse eggs were obtained by providing actively laying adult lice with a closed mesh nylon gauze substrate over a 48 hour period. At the end of this period the lice were removed and the gauze cut into smaller pieces each of which was allocated to a respective Petri dish.

In the test procedure 5-10 ml of a test solution was poured into a the base of a 3 ml dish, and gauze bearing lice or louse eggs was immersed in the test solution for about 10 sec. during which the gauze was turned at least twice to remove air bubbles. After removal from the test solution the gauze was blotted to remove excess fluid and returned to another dish.

Gauze squares bearing the lice or eggs were incubated at 30°C± 2° and at 50% ± 15% relative humidity for times of 10 min and 2 hours. At the end of the selected time period the gauze was washed using a bland toiletry shampoo diluted one part shampoo to nine parts water (FWS1:9) and rinsed three times using 250 ml of warm tap water (34°C). The gauze is then blotted dry using a medical tissue wipe and incubated under normal conditions until the results are read. The mortality of lice was recorded after 24 hours and for louse eggs after 10 days once the control group had hatched.

The control batches were tested using 60% IPA solution.

**Table 1**

| ( Lice ) | | | | | |
|---|---|---|---|---|---|
| sample | time | dead | alive | morbid | mortality |
| 1 | 10 min | 6 | 13 | 1 | 35% |
| | 2 hrs | 10 | 4 | 4 | 77.8% |
| 2 | 10 min | 7 | 11 | 1 | 42.1% |
| | 2 hrs | 9 | 9 | 3 | 57.1 |
| 3 | 10 min | 4 | 16 | 2 | 27.3% |
| | 2 hrs | 10 | 7 | 1 | 61.1% |
| 4 | 10 min | 3 | 17 | 1 | 19% |
| | 2 hrs | 17 | 0 | 3 | 100% |
| control | 10 min | 2 | 17 | 0 | 10% |
| | 2 hrs | 4 | 14 | 1 | 26% |

**Table 2**

| (eggs ten minutes exposure) | | | | | |
|---|---|---|---|---|---|
| sample | hatched | half-hatched | undeveloped | dead | % mortaliy |
| 1 | 164 | 3 | 21 | 4 | 13.0 |
| 2 | 113 | 0 | 21 | 11 | 22.0 |
| 3 | 93 | 0 | 32 | 26 | 38.4 |
| 4 | 99 | 2 | 13 | 6 | 15.8 |
| control | 94 | 0 | 9 | 1 | 9.6 |

**Table 3**

| (eggs 2 hours exposure) | | | | | |
|---|---|---|---|---|---|
| sample | hatched | half-hatched | undeveloped | dead | % mortality |
| 1 | 129 | 6 | 30 | 4 | 20.1 |
| 2 | 147 | 5 | 41 | 5 | 23.2 |
| 3 | 2 | 1 | 92 | 13 | 97.2 |
| 4 | 117 | 6 | 54 | 36 | 42.3 |
| control | 148 | 0 | 14 | 8 | 12.9. |

The test results in table 1 show that sample 2 is the most effective against lice with a 10 minute exposure whilst a 2 hour exposure it did not greatly improve its efficacy. Sample 4 had a very low level of mortality at 10 minutes exposure but was 100% effective after 2 hours

Table 2 shows that the samples after 10 minutes exposure have little effect on louse eggs except for sample 3.

Table 3 shows that the samples have an increase in mortality at 2 hours exposure. Formulations 3 and 4 have greatly increased mortality rates at 2 hours exposure.

Sample 5, was similarly tested and observations made relating to the mortality of lice after 24 hours and of louse eggs when the control group has completed emergence after 10 days. The results are shown in table 4 & 5 below.

**Table 4**

| (2 hours exposure against adult lice) | | | | |
|---|---|---|---|---|
| sample | dead | alive | morbid | mortality |
| 5 | 10 | 5 | 3 | 72% |
| control | 1 | 19 | 0 | 5% |

**Table 5**

| (2 hours exposure against louse eggs) | | | | | |
|---|---|---|---|---|---|
| sample | hatched | half-hatched | undeveloped | dead | mortality |
| 5 | 1 | 0 | 296 | 1 | 99.7% |
| control | 120 | 6 | 10 | 10 | 17.8 |

This sample showed good efficacy against both adult lice and lice eggs.

## Claims

1. An aqueous composition for the treatment of headlice and their eggs and which includes as its active ingredients at least one essential oil **characterised in that** the composition further includes an infusion of: dried peppermint leaves, a tea, and garlic.

2. A composition as claimed in claim 1 wherein the infusion of tea is an infusion of a mixture of tea and oswego tea (bergamot) commonly known as Earl Gray tea.

3. A composition as claimed in Claim 1 or Claim 2 and further including as an essential oil at least one of citronella oil, tea tree oil, eucalyptus oil.

4. A composition as claimed in claim 3, wherein the essential oil comprises a 50/50 mixture of tea tree oil with one of citronella oil and eucalyptus oil.

5. A composition as claimed in Claim 3 or Claim 4 , wherein the essential oil comprises less than 3% of the total composition by weight.

6. A composition as claimed in any one of Claims 1 to 5, wherein the active ingredients infused into the water comprise less than 0.5% of the total composition by weight.

7. A composition as claimed in any one of Claims 1 to 6, wherein the composition further comprises acetic acid present up to a maximum of 2.5% by weight.

8. A composition as claimed in any one of Claims 1 to 7 and further comprising surfactants.

9. A composition as claimed in any one of Claims 1 to 8 wherein and further comprising a thickening agent selected from carboxymethylcellulose, a polyglycol, and guam, typically up to 1% by weight.

10. A method of manufacture of a composition for the treatment of headlice including the steps of:
making an infusion of peppermint leaves, tea and garlic in boiling water and allowing to cool, adding essential oils to the cooled infusion and then mixing the cooled infusion with surfactants and thickening agents to form a gel.

11. A method as claimed in Claim 10, wherein the essential oil comprises at least two oils mixed before addition to the cooled infusion.

12. A method as claimed in Claim 10 or Claim 11, wherein an aqueous solution of acetic acid is added to the cooled infusion with the essential oil, and any surfactants and thickening agents.

13. Use of a composition as claimed in any one of claims 1 to 9 for the manufacture of a hair cleaning agent for the treatment of headlice and/or erradication of their eggs.

## Patentansprüche

1. Wässrige Zusammensetzung für die Behandlung von Kopfläusen und ihren Eiern, und welche als ihre aktiven Bestandteile mindestens ein etherisches Öl einschließt, **dadurch gekennzeichnet, daß** die Zusammensetzung weiterhin einen Aufguß von: getrockneten Pfefferminzblättern, einem Tee und Knoblauch einschließt.

2. Zusammensetzung nach Anspruch 1, wobei der Aufguß von Tee ein Aufguß eines Gemisches von Tee und Oswego-Tee (Bergamotte), gemeinhin bekannt als Earl Gray Tee, ist.

3. Zusammensetzung nach Anspruch 1 oder Anspruch 2 und weiterhin einschließend als etherisches Öl mindestens eines von Citronellaöl, Teebaumöl, Eukalyptusöl.

4. Zusammensetzung nach Anspruch 3, wobei das etherische Öl ein 50/50-Gemisch von Teebaumöl mit einem von Citronellaöl und Eukalyptusöl umfaßt.

5. Zusammensetzung nach Anspruch 3 oder Anspruch 4, wobei das etherische Öl weniger als 3 Gew.-% der Gesamtzusammensetzung umfaßt.

6. Zusammensetzung nach einem der Ansprüche 1 bis 5, wobei die aktiven Bestandteile, aufgegossen im Wasser, weniger als 0,5 Gew.-% der Gesamtzusammensetzung umfassen.

7. Zusammensetzung nach einem der Ansprüche 1 bis 6, wobei die Zusammensetzung weiterhin Essigsäure, vorhanden bis zu einem Maximum von 2,5 Gew.-%, umfaßt.

8. Zusammensetzung nach einem der Ansprüche 1 bis 7 und weiterhin umfassend Tenside.

9. Zusammensetzung nach einem der Ansprüche 1 bis 8, worin und weiterhin umfassend ein Verdickungsmittel, ausgewählt aus Carboxymethylcellulose, einem Polyglycol und Guam, typischerweise bis zu 1 Gew-%.

10. Verfahren für die Herstellung einer Zusammensetzung für die Behandlung von Kopfläusen, einschließend die Schritte:
Herstellen eines Aufgusses von Pfefferminzblättern, Tee und Knoblauch in siedendem Wasser und Abkühlenlassen, Hinzufügen von etherischen Ölen zu dem abgekühlten Aufguß und dann Mischen des abgekühlten Aufgusses mit Tensiden und Verdickungsmitteln, um ein Gel zu erzeugen.

11. Verfahren nach Anspruch 10, wobei das etherische Öl mindestens zwei Öle, gemischt vor der Zugabe zu dem abgekühlten Aufguß, umfaßt.

12. Verfahren nach Anspruch 10 oder Anspruch 11, wobei eine wässrige Lösung von Essigsäure zu dem abgekühlten Aufguß mit dem etherischen Öl und allen Tensiden und Verdickungsmitteln hinzugegeben wird.

13. Verwendung einer Zusammensetzung nach einem der Ansprüche 1 bis 9 für die Herstellung eines Haarreinigungsmittels für die Behandlung von Kopfläusen und/oder Vernichtung ihrer Eier.

## Revendications

1. Composition aqueuse pour le traitement des poux et de leurs oeufs et qui inclut comme ingrédients actifs au moins une huile essentielle **caractérisée en ce que** la composition inclut de plus une infusion : de feuilles de menthe poivrée séchées, d'un thé, et d'ail.

2. Composition selon la revendication 1 dans laquelle l'infusion de thé est une infusion d'un mélange de thé et de thé d'Oswego (bergamote) communément appelé thé Earl Gray.

3. Composition selon la revendication 1 ou la revendication 2 et incluant de plus comme huile essentielle au moins l'une des huiles de citronnelle, d'arbre à thé, d'eucalyptus.

4. Composition selon la revendication 3, dans laquelle l'huile essentielle comprend un mélange à 50/50 d'huile d'arbre à thé avec l'une des huiles de citronnelle et d'eucalyptus.

5. Composition selon la revendication 3 ou la revendication 4, dans laquelle l'huile essentielle comprend moins de 3 % en poids de la composition totale.

6. Composition selon l'une quelconque des revendications 1 à 5, dans laquelle les ingrédients actifs infusés dans l'eau comprennent moins de 0,5 % en poids de la composition totale.

7. Composition selon l'une quelconque des revendications 1 à 6, dans laquelle la composition comprend de plus de l'acide acétique présent jusqu'à un maximum de 2,5 % en poids.

8. Composition selon l'une quelconque des revendications 1 à 7 et comprenant de plus des surfactants.

9. Composition selon l'une quelconque des revendications 1 à 8 dans laquelle et comprenant de plus un agent épaississant sélectionné parmi de la carboxyméhtylcellulose, un polyglycol, et de la gomme de guar, typiquement jusqu'à 1 % en poids.

10. Procédé de fabrication d'une composition pour le traitement des poux incluant les étapes :
de fabrication d'une infusion de feuilles de menthe poivrée, de thé et d'ail dans de l'eau bouillante et de permission du refroidissement, d'ajout d'huiles essentielles à l'infusion refroidie et ensuite du mélange de l'infusion refroidie avec des surfactants et des agents épaississant pour former un gel.

11. Procédé selon la revendication 10, dans lequel l'huile essentielle comprend au moins deux huiles mélangées avant addition de l'infusion refroidie.

12. Procédé selon la revendication 10 ou la revendication 11, dans lequel une solution aqueuse d'acide acétique est ajoutée à l'infusion refroidie avec l'huile essentielle, et de quelconques surfactants et agents épaississants.

13. Utilisation d'une composition comme revendiquée selon l'une quelconque des revendications 1 à 9 pour la fabrication d'un agent de nettoyage de cheveux pour le traitement des poux et/ou l'éradication de leurs oeufs.
